# EUROPEAN PATENT APPLICATION

(11) **EP 4 582 109 A1**
(43) Date of publication of application: **09.07.2025**
(21) Application number: 23813258.3
(22) Date of filing: 31.10.2023
(51) Int. Cl.: A61K 51/06, A61K 51/12, A61P 1/16, A61P 35/00, A61K 103/32

(54) **YTTRIUM [90Y] MICROSPHERE, PREPARATION METHOD THEREFOR, AND USE THEREOF**

(30) Priority: 25.10.2023 CN 202311388190
(71) Applicant: Mednovo Group Co. Ltd, Suzhou, Jiangsu 215163 (CN)
(72) Inventor: LI, Peishang, Suzhou, Jiangsu 215163 (CN); KONG, Xiangsheng, Suzhou, Jiangsu 215163 (CN); WU, Jian, Suzhou, Jiangsu 215163 (CN); LUO, Chentao, Suzhou, Jiangsu 215163 (CN)
(74) Representative: J A Kemp LLP
(86) International application number: PCT/CN2023/128145
(87) International publication number: WO 2025/086316

(57) **Abstract**

The present application relates to the technical field of radiopharmaceutical preparation, specifically relates to an yttrium [⁹⁰Y] microsphere and preparation method therefor and use thereof. The yttrium [⁹⁰Y] microsphere comprises a polystyrene sulfonic acid type resin microsphere and yttrium-90 adsorbed within the polystyrene sulfonic acid type resin microsphere. The use of polystyrene sulfonic acid type resin microsphere to adsorb yttrium-90 has significantly improved adsorption strength, significantly improved safety during preparation and use, and has a good killing effect on tumor cells.

## Description

### CROSS REFERENCE TO RELATED APPLICATIONS

The present application claims priority to Chinese Patent Application No. 202311388190.4, entitled "YTTRIUM [90Y] MICROSPHERE AND PREPARATION METHOD THEREFOR AND USE THEREOF", and filed to the China National Intellectual Property Administration on October 25, 2023, the entire contents of which are incorporated herein by reference.

### TECHNICAL FIELD

The present application relates to the technical field of radiopharmaceutical preparation, specifically relates to an yttrium [⁹⁰Y] microsphere and preparation method therefor and use thereof.

### BACKGROUND

Interventional radiotherapy technology is an effective method for treating tumor developed in recent years. Yttrium [⁹⁰Y] as a radiotherapy has the following advantages: high energy, short half-life, short penetration distance in the body, and easy protection. Yttrium [⁹⁰Y] microspheres injection precision interventional radiotherapy is a technology that integrates interventional and precision internal radiation therapy. Yttrium [⁹⁰Y] radioactive microspheres, as an important treatment for liver cancer, will hopefully provide a powerful weapon for the comprehensive treatment of liver cancer patients in our country, which can improve their cure rate and prolong the overall survival.

Yttrium-90 glass microspheres and styrene divinylbenzene copolymer microspheres have been used for the treatment of liver cancer. However, these microspheres have the defect of insufficient adsorption strength for radioactive elements, resulting in poor therapeutic effect on tumors.

### SUMMARY OF THE INVENTION

Therefore, the technical problem to be solved in the present application is to overcome the defect of insufficient adsorption strength of yttrium [⁹⁰Y] microspheres in the prior art, so as to provide an yttrium [⁹⁰Y] microspheres and preparation method therefor and use thereof.

For this purpose, the present application provides an yttrium [⁹⁰Y] microsphere, wherein the yttrium [⁹⁰Y] microsphere comprises a polystyrene sulfonic acid type resin microsphere and yttrium-90 adsorbed within the polystyrene sulfonic acid type resin microsphere.

Further, the polystyrene sulfonic acid type resin microsphere is prepared by reacting a polystyrene microsphere with a sulfonating agent.

Further, the sulfonating agent is selected from one or more of liquid sulfur trioxide, concentrated sulfuric acid, chlorosulfonic acid, or sulfite; preferably, the sulfonating agent is concentrated sulfuric acid.

Wherein, the mass percentage of concentrated sulfuric acid in the present application is greater than or equal to 70%, such as 98%.

Further, the polystyrene microsphere is prepared by using styrene as a monomer and divinylbenzene as a crosslinking agent, mixing and reacting with an aqueous solution containing a suspending agent in the presence of an initiator and pore forming agent.

Further, the pore forming agent is selected from one or more of toluene and n-heptane; and/or, the suspension agent is polyvinyl alcohol; and/or, the initiator is benzoyl peroxide.

Further, the preparation method of the polystyrene sulfonic acid type resin microsphere comprises:
preparation of polystyrene microspheres: mixing styrene, divinylbenzene, initiator, and pore forming agent, dissolving them to obtain an oil phase, injecting the oil phase into an aqueous solution containing a suspension agent, shearing emulsification, and heating reaction to obtain the polystyrene microspheres; and
preparation of polystyrene sulfonic acid type resin microspheres: adding polystyrene microspheres to concentrated sulfuric acid, ultrasonic dispersing, and heating reaction to obtain the polystyrene sulfonic acid type resin microspheres.

Further, during the preparation of polystyrene microspheres, the temperature of heating reaction is 80 °C to 100 °C, and the time is 2 hours to 5 hours. Stirring is performed at 40°C to 60°C overnight before the heating reaction. The time of shearing emulsification is 3 min to 10 min.

Further, during the preparation of polystyrene sulfonic acid type resin microspheres, adding polystyrene microspheres to concentrated sulfuric acid at 0°C to 4°C, and heating to 37°C to 45°C for reaction.

Further, the heating reaction also comprises the steps of suction filtration, washing, drying and sieving.

Further, the polystyrene microsphere is made of the following raw materials:
an aqueous solution containing polyvinyl alcohol of 100 parts by volume;
syrene of 2.5 to 5.5 parts by volume;
divinylbenzene of 1.2 to 4.2 parts by volume;
benzoyl peroxide of 0.02-2.02 parts by weight;
toluene of 0.6 to 1.6 parts by volume; and
n-heptane of 0-1.6 parts by volume;
wherein the ratio between parts by weight and parts by volume is g/mL.

Further, the yttrium [⁹⁰Y] microsphere has a radioactive activity ranging from 2.5 GBq/g to 25 GBq/g.

Further, the mass percentage of polyvinyl alcohol in the aqueous solution containing polyvinyl alcohol is in a range from 0.5% to 3% (e.g., 1%, 2%, or 0.8%).

Further, the yttrium [⁹⁰Y] sulfonic acid type resin microsphere has a particle size ranging from 10µM to 60µM, preferably 28µm to 35µm, more preferably 30µm to 35µm.

The present application also provides a method for preparing the yttrium [⁹⁰Y] microsphere above, wherein the method comprises:
S1. mixing an yttrium chloride [⁹⁰Y]solution and yttrium sulfate-89 solution with polystyrene sulfonic acid type resin microspheres; and
S2. washing with a buffer salt solution and water to obtain an yttrium[⁹⁰Y] microsphere.

Further, the ratio of radioactive activity of yttrium chloride [⁹⁰Y] solution, the volume of yttrium sulfate-89 solution, and the mass of polystyrene sulfonic acid type resin microspheres is 30-200GBq: 30-200 ml: 5-15.5 g. Preferably, the concentration of yttrium sulfate-89 solution is in a range from 1g/ L to 9.5g/ L.

Wherein, the yttrium chloride [⁹⁰Y] solution has a concentration ranging from 1 mg/ml to 5mg/ml.

Further, the ratio of the volume of buffer salt solution and the mass of polystyrene sulfonic acid type resin microspheres is 70-300ml: 5-15.5g.

Further, the buffer salt has a concentration ranging from 2 g/l to 60g/l.

Further, the buffer salt solution is selected from one or more of sodium phosphate solution, sodium dihydrogen phosphate solution, disodium hydrogen phosphate solution, potassium phosphate solution, potassium dihydrogen phosphate solution, dipotassium hydrogen phosphate solution, PBS solution, sodium acetate and Tris-HCl buffer solution. Optionally, in the S2 step, sodium phosphate solution is used first for washing, and then sodium dihydrogen phosphate solution is used for washing.

Further, in the S2 step, water, sodium phosphate solution, sodium dihydrogen phosphate solution, and water are used sequentially for washing.

Further, the sodium dihydrogen phosphate solution has a concentration ranging from 2 g/L to 25g/L; the sodium phosphate solution has a concentration ranging from 10 g/L to 60g/L.

The present application also provides a pharmaceutical preparation of yttrium [⁹⁰Y] microsphere, comprising any one of the yttrium [⁹⁰Y] microsphere above or yttrium [⁹⁰Y] microsphere prepared by any one of the methods above, and also a pharmaceutically acceptable excipient. Optionally, the pharmaceutical acceptable excipient is a solvent.

Wherein, the solvent can be distilled water, water for injection, or glucose aqueous solution and other conventional solvents. For the pharmaceutical preparation of yttrium [⁹⁰Y] microspheres, in the unit dose, the radioactive activity of yttrium [⁹⁰Y] microspheres is ranging from 3 GBq to 20GBq.

Further, the pharmaceutical preparation is an injection, and the radioactive activity of the injection per unit dose is ranging from 3 GBq to 20GBq.

The unit dose of the injection comprises 0.8g to 4g of the yttrium [⁹⁰Y] microspheres.

The preparation method of yttrium [⁹⁰Y] microspheres injection in the present application can also comprise routine intermediate radioactivity detection, encapsulation, sterilization or product activity detection.

Wherein, the filling volume of each injection bottle is 5 ml when encapsulation.

The present application also provides the use of any of the above-mentioned yttrium [⁹⁰Y] microspheres or yttrium [⁹⁰Y] microspheres prepared by any one of the above-mentioned preparation methods or any of the above-mentioned pharmaceutical preparations in the preparation of medicaments for preventing or treating liver cancer.

The raw material (yttrium chloride [⁹⁰Y] solution) is commercially available, and its radioactive activity can also be customized. According to the activity meter operation instructions, measure and record the activity of incoming raw materials (yttrium chloride [⁹⁰Y] solution) to ensure that the activity of radioactive raw materials is consistent with the target activity during production. The formula for calculating the activity of the required radioactive raw material is: A=kA0/e^{-0.26t}, wherein k is the process loss coefficient, A0 is the activity value at the calibration time, A is the radioactive activity value at the determination time, and t is the difference in days between the determination time and the calibration time.

The technical solution of the present application has the following advantages:
1. The yttrium [⁹⁰Y] microsphere provided in the present application comprises a polystyrene sulfonic acid type resin microsphere and yttrium-90 adsorbed within the polystyrene sulfonic acid type resin microsphere. The use of polystyrene sulfonic acid type resin microsphere to adsorb yttrium-90 has significantly improved adsorption strength, significantly improved safety during preparation and use, and good killing effect on tumor cells.
2. The yttrium [⁹⁰Y] microspheres provided in the present application can significantly improve the killing effect on liver cancer by controlling the particle size of polystyrene sulfonic acid resin microspheres to be in a range from 10µm to 60 µm, preferably 28µm to 43 µm, more preferably 30µm to 35 µm.
   The microsphere with a particle size 32.5µm ± 2.5µm has a density ranging from 1.125g/ml to 1.6g/ml in water (equivalent to red blood cells) and can withstand moist heat sterilization.
3. The yttrium [⁹⁰Y] microspheres provided in the present application comprises an aqueous solution containing polyvinyl alcohol of 100 parts by volume; syrene of 2.5 to 5.5 parts by volume; divinylbenzene of 1.2 to 4.2 parts by volume; benzoyl peroxide of 0.02-2.02 parts by weight; toluene of 0.6 to 1.6 parts by volume; and n-heptane of 0 to 1.6 parts by volume. By controlling the ratio of various substances in the prescription within the optimal range, the polystyrene sulfonic acid type resin microspheres obtained have significantly increased ion capacity.
4. The yttrium [⁹⁰Y] microsphere injection provided in the present application comprises the yttrium [⁹⁰Y] microsphere in the present application. Polystyrene sulfonic acid type resin microsphere is used to adsorb yttrium 90, and less yttrium ions are precipitated. The safety during preparation and use is significantly improved, and the killing effect on tumor cells is good.
5. In the yttrium [⁹⁰Y] microsphere injection provided in the present application, the resin microsphere is first washed with sodium phosphate solution, and then washed with sodium dihydrogen phosphate solution in the S2 step. The combination with yttrium ions is better, the precipitation of yttrium ions is less, and the safety is significantly improved.

### DETAILED DESCRIPTION

The following embodiments are provided for a better understanding of the present invention but not for limiting the content and protection scope of the present invention. Any product identical or similar to the present invention obtained by combining with the features of other prior art shall fall within the protection scope of the present invention.

If the specific experimental steps or conditions are not indicated in the examples, it can be carried out according to the operations or conditions of the conventional experimental steps described in the literature in this field. The reagents or instruments used without the manufacturer's indication are all conventional reagent products that can be obtained from commercial sources.

In the present application, liquid can be discharged through conventional commercially available filters, such as filters from manufacturers such as Merck Millipore or Steritech in the United States, model TKT04700/PCT12047100, polycarbonate, filter aperture 10µm to 18 µm. The flow rate is 15-65ml/min.

The process for preparing yttrium [⁹⁰Y] microsphere injection comprises the following steps:
transferring microspheres: adding water for injection to the polystyrene sulfonic acid type resin microspheres to form a suspension and transferring it to a reaction vessel.
adding yttrium solution: flowing the yttrium sulfate-89 solution through a delivery vial containing yttrium chloride [⁹⁰Y] solution to transfer the yttrium chloride [⁹⁰Y] solution and yttrium sulfate-89 solution together into a reaction vessel, stirring and mixing for at least 10 minutes (e.g. 10 to 50 minutes), and discharging the liquid through a filter.
first washing microspheres with water: adding water for injection to the reaction vessel, stirring and mixing for at least 1 minute (e.g. 5 to 20 minutes); filtering and discharging the liquid, and repeating this step more than once.
washing with buffer salt: adding a buffer salt solution to the reaction vessel, stirring and mixing for at least 5 minutes, filtering and discharging the liquid.
washing with water: injecting 100 ml of water for injection into the reaction vessel, stirring and mixing for at least 1 minute (e.g. 10 minutes), filtering and discharging the liquid; repeating this step to prepare yttrium [⁹⁰Y] microspheres, injecting water for injection, and discharging the liquid by filtering, thereby the solution in the reaction vessel achieving the target volume, thus obtaining yttrium [⁹⁰Y] microsphere injection.

### Example 1

This example provides a method for preparing polystyrene sulfonic acid type resin microspheres, using the following reagents: polyvinyl alcohol (PVA), styrene (St), divinylbenzene (DVB), toluene, n-heptane, ethanol, and benzoyl peroxide (BPO).

The preparation method is as follows:
S1: St and DVB were first washed with a 10% sodium hydroxide aqueous solution once, and then washed with water three times for later use.
S2: PVA was heated and dissolved in water to prepare a 100ml of 1% (mass percentage) PVA aqueous solution, and protected it with nitrogen gas to obtain the aqueous phase. 2.5ml of St, 1.2ml of DVB, 0.02g of BPO, 0.6ml of toluene and 0.6ml of n-heptane were weighted and mixed, and dissolved to obtain the oil phase. The oil phase was injected into the water phase and sheared for emulsification for 5 minutes using a shear machine. Stirred at 50°C overnight and reacted at 90°C for 3 hours to obtain polystyrene microspheres. The polystyrene microspheres were filtered, washed twice with water, washed once with ethanol, and dried.
S3: The dried polystyrene microspheres were added to 25mL of concentrated sulfuric acid (98% by mass) at 0°C, and then dispersed under ultrasound and reacted overnight at 40°C. Filtered, washed to neutral, beat twice with hot water at 95°C, washed once with ethanol, and dried.
S4: Sieved and collected polystyrene sulfonic acid type resin microspheres with a particle size ranging from 30µm to 35µm.

This example also provides an yttrium [⁹⁰Y] microsphere injection and preparation method therefor. The prescription dosage of 10 bottles of injection was shown in Table 1:

**Table 1 Prescription Table**

| Name | Dosage |
|---|---|
| 5mg/mL yttrium chloride [⁹⁰Y] solution | 60GBq |
| Polystyrene sulfonic acid type resin microsphere | 11.5 g |
| Yttrium oxide-89 powder | 0.3 g |
| 0.1M sulfuric acid | 80 ml |
| 30g/L sodium phosphate solution | 120 ml |
| 10g/L sodium dihydrogen phosphate solution | 150 ml |
| Water for injection | q.s. |

Wherein, preparation of yttrium sulfate-89 solution: 0.3g of yttrium oxide-89 powder was dissolved in 80ml of 0.1 M sulfuric acid at a temperature of 40°C to obtain an yttrium sulfate-89 solution, and place it in a production reserve container for future use.

The preparation method is as follows:
(1) Microspheres transfer: 100mL of water for injection was added to the polystyrene sulfonic acid type resin microspheres to form a suspension, and all the microspheres were transferred to the reaction vessel.
(2) Adding yttrium solution: the yttrium sulfate-89 solution was transferred into a reaction vessel through a transfer vial containing yttrium chloride [⁹⁰Y] solution, in order to transfer the yttrium chloride [⁹⁰Y] solution and yttrium sulfate-89 solution together into the reaction vessel. Stirred and mixed for 10 minutes, and discharged the liquid by filtration.
(3) First washing microspheres with water: 100 ml of water for injection was added to the reaction vessel, stirred and mixed for 1 minute; filtered and discharged the liquid and repeated this step twice.
(4) Washing microsphere with phosphate: 120 ml of 30g/L sodium phosphate solution was added to the reaction vessel, stirred and mixed for 10 minutes, and discharged the liquid by filtration. Then, 150ml of 10g/L sodium dihydrogen phosphate solution was added, stirred and mixed for 5 minutes, and discharged the liquid by filtration.
(5) Second washing microspheres with water: 100 ml of water for injection was injected to the reaction vessel, stirred and mixed for 1 minute; filtered and discharged the liquid; washed repeatedly twice in this way. 100 ml of water for injection was injected and discharged the liquid by filtration to reach a volume of 50 ml in the reaction vessel.
(6) Encapsulation: the solution was distributed into injection bottles, each with a capacity of 5mL, plugged with a rubber stopper, and rolled the edge and cap. This step was controlled by an automated system, which involved automatic filling and capping.
(7) Sterilization: 132°C, 7 minutes.

The radioactive activity of yttrium [⁹⁰Y] microsphere injection was determined to be 6GBq/bottle using the radioactive activity measurement method (General Rule 1401 of Part Four of the Pharmacopoeia of the People's Republic of China 2020 Edition).

### Example 2

This example provides a method for preparing polystyrene sulfonic acid type resin microspheres, which is substantially the same as the raw materials and process of Example 1, with the only difference being that the collected polystyrene sulfonic acid type resin microspheres with a particle size ranging from 28µm to 33µm after sieving in Step S4.

This example provides an yttrium [⁹⁰Y] microsphere injection and preparation method therefor, which is substantially the same as the raw materials and process of Example 1, with the only difference being that the polystyrene sulfonic acid type resin microsphere prepared in this example was used to replace the polystyrene sulfonic acid type resin microsphere in Example 1.

### Example 3

This example provides a method for preparing polystyrene sulfonic acid type resin microspheres, which is substantially the same as the raw materials and process of Example 1, with the only difference being that the collected polystyrene sulfonic acid type resin microspheres with a particle size ranging from 38µm to 43µm after sieving in Step S4.

This example provides an yttrium [⁹⁰Y] microsphere injection and preparation method therefor, which is substantially the same as the raw materials and process of Example 1, with the only difference being that the polystyrene sulfonic acid type resin microsphere prepared in this example was used to replace the polystyrene sulfonic acid type resin microsphere in Example 1.

### Example 4

This example provides a method for preparing polystyrene sulfonic acid type resin microspheres, which is substantially the same as the raw materials and process of Example 1, with the only difference being that the collected polystyrene sulfonic acid type resin microspheres with a particle size ranging from 13 µm to 18 µm after sieving in Step S4.

This example provides an yttrium [⁹⁰Y] microsphere injection and preparation method therefor, which is substantially the same as the raw materials and process of Example 1, with the only difference being that the polystyrene sulfonic acid type resin microsphere prepared in this example was used to replace the polystyrene sulfonic acid type resin microsphere in Example 1.

### Example 5

This example provides a method for preparing polystyrene sulfonic acid type resin microspheres, which is substantially the same as the raw materials and process of Example 1, with the only difference being that the collected polystyrene sulfonic acid type resin microspheres with a particle size ranging from 48µm to 53µm after sieving in Step S4.

This example provides an yttrium [⁹⁰Y] microsphere injection and preparation method therefor, which is substantially the same as the raw materials and process of Example 1, with the only difference being that the polystyrene sulfonic acid type resin microsphere prepared in this example was used to replace the polystyrene sulfonic acid type resin microsphere in Example 1.

### Experiment Example 1

To investigate the effects of different pore forming agent and crosslinking agents during the preparation process of polystyrene sulfonic acid type resin microspheres as well as their amounts on the ion exchange capacity of polystyrene sulfonic acid resin microspheres, the preparation method was substantially the same as Example 1, with the only difference being the composition of the oil phase, as shown in the table below. The ion exchange capacity of polystyrene sulfonic acid resin microspheres prepared using the following groups A to C method and commercially available microspheres were tested.

The specific method was as follows: 100ml of purified water was added to an appropriate amount of polystyrene sulfonic acid resin microspheres, stirred for 1 minute, washed repeatedly 3 times, and filtered. The filtered resin microspheres were weighted and recorded as M. 500ml of 0.02M sodium hydroxide solution was added to the microspheres for immersion and then sonicated for 20 minutes. Filtered by suction, 100ml of the filtrate was taken, two drops of phenolphthalein was added and then performed a titration experiment with 0.01M hydrochloric acid. The volume of hydrochloric acid used was recorded at the titration endpoint, recorded as V.

The calculation formula for ion exchange capacity was as follows: ion exchange capacity (mmol/g) = (molar concentration of sodium hydroxide solution × volume of sodium hydroxide - molar concentration of hydrochloric acid × V) /M.

**Table 2 Experimental Design**

| Name | Oil phase preparation method for self-made products or information on commercially available products |
|---|---|
| A | 2.5ml of St, 1.2ml of DVB, 0.02g of BPO, 0.6ml of toluene, and 0.6ml of n-heptane were weighted, mixed and dissolved to obtain the oil phase |
| B | 3.5ml of St, 4.2ml of DVB, 1.02g of BPO and 0.8ml of toluene were weighted, mixed and dissolved to obtain the oil phase |
| C | 5.5ml of St, 3.2ml of DVB, 2.02g of BPO, 1.6ml of toluene, and 1.6ml of n-heptane were weighted, mixed and dissolved to obtain the oil phase |
| D | Ion exchange chromatography medium Nano, purchased from Changshu Nano Microbiology Technology Co., Ltd. Resin microspheres, specification: 300mL |
| E | Ion exchange chromatography medium NanoGel, purchased from Changshu Nano Microbiology Technology Co., Ltd. specification: 300mL |
| F | Ion exchange filler Proteomix POR30-S: Suzhou Saifen Technology Co., Ltd., specification: 300 ml |

**Table 3 Results of ion exchange capacity**

| Name | 0.02M sodium hydroxide solution (ml) | 0.01M volume of hydrochloric acid titrated ( (ml) | M (g) | Ion exchange capacity (mmol/g) |
|---|---|---|---|---|
| A | 500 | 17.01 | 1.0039 | 9.79 |
| B | 500 | 17.01 | 1.2223 | 8.04 |
| C | 500 | 17.10 | 1.617 | 6.08 |
| D | 500 | 17.03 | 2.0073 | 4.90 |
| E | 500 | 17.03 | 2.2223 | 4.42 |
| F | 500 | 17.02 | 1.8502 | 5.31 |

From the above table, it can be seen that compared to commercially available products, the ion exchange capacity of the resin microspheres prepared in Groups A to C of the present application is significantly improved, especially group A, which used the same method as Example 1, showed significant improvement compared to both group B and group C.

### Experiment Example 2: A Nude Mouse Experiment for Treating Primary Hepatocellular Liver Cancer

### 1. Test method

Experimental nude mice weighing 20g ± 2g, all female, each was inoculated with 3×10⁶ SMMC-7721 cells, when the tumor grew to 5mm to 10mm, they were randomly divided into experimental groups 1 to 5 and control group, with 5 mice in each group, and treatment was started. 1mL of the injection prepared in Examples 1 to 5 were taken, diluted with water to a constant volume of 10mL to obtain the test drug solutions for each group. Experimental groups 1 to 5 animals were injected intraperitoneally with the test drug solution (5ml/ animal) prepared in Examples 1 to 5, once daily for 10 consecutive days. The control group was given the same volume of water for injection and observed for 10 days. After 10 days of discontinuation, all animals were euthanized 24 hours after discontinuation, tumor masses were dissected, tumor weight was measured, and tumor inhibition rate was calculated according to the following formula: Tumor inhibition rate = (tumor weight of control group - tumor weight of experimental group/ tumor weight of control group) × 100%.

### 2. Test results:

**Table 4 Tumor inhibition rates of microspheres with different particle sizes on nude mice with liver cancer.**

| Item | Number of nude mice at the beginning of the experiment | Number of nude mice at the end of the experiment | Tumor weight (x±SD) | Inhibition rate % |
|---|---|---|---|---|
| Control group | 5 | 5 | 6.33±0.02 | |
| Example 1 | 5 | 5 | 0.06±0.06 | 99.05 |
| Example 2 | 5 | 5 | 0.21±0.61 | 96.68 |
| Example 3 | 5 | 5 | 0.33+0.74 | 94.79 |
| Example 4 | 5 | 5 | 1.26±0.46 | 80.09 |
| Example 5 | 5 | 5 | 1.44±0.46 | 77.25 |

From the above table, it can be seen that the microspheres prepared in Examples 1 to 3 has a much better killing effect on tumor cells than the microspheres prepared in Examples 4 and 5. Example 1 is the best, followed by Example 2.

### Experiment Example 3

To investigate the effects of using different phosphates as washing agent on the encapsulation efficiency yttrium [⁹⁰Y] microspheres and dissolution rate of yttrium, the same method as Example 1 was used to prepare yttrium [⁹⁰Y] microsphere injection. The difference only lies in the different types of phosphates in step (4) (in addition, the concentration of yttrium chloride [⁹⁰Y] solution added to group D and group E is different). Group A all followed Example 1, that is, washing with sodium phosphate solution first and then washing with sodium dihydrogen phosphate solution, the specific operations of step (4) in Group A and other groups are shown in Table 5 below, and the other steps are the same as Example 1.

All the filtrate generated during the preparation process of yttrium [⁹⁰Y] microsphere injection prepared by each group (including the steps of adding yttrium solution, first washing microspheres with water, washing microsphere with phosphate, and second washing with water) were collected and test solution 1 was obtained after 0.45µm filtration. The concentration of yttrium ions in test solution 1 was measured by inductively coupled plasma mass spectrometer (recorded as yttrium ions in the filtrate).

The preservation solution of yttrium [⁹⁰Y] microspheres prepared in each group was filtered out and test solution 2 was obtained after 0.45µm filtration. The concentration of yttrium ions in test solution 2 was measured by inductively coupled plasma mass spectrometer (recorded as yttrium ions precipitated).

Preparation of standard curve: 50 µL of Y89 standard solution was transferred into a 10ml volumetric flask, and volume with 2% nitric acid to obtain a 5mg/L intermediate solution. 0.02ml, 0.1ml, 0.2ml, 0.4ml, and 1.0ml of the intermediate solution were transferred to different 10ml volumetric flasks, 20 µL Ge internal standard with a concentration of 10mg/L was added, and volume with 2% nitric acid to obtain a series of standard solutions with Y⁸⁹ concentrations of 10 µg/L, 50 µg/L, 100 µg/L, 200 µg/L, and 500 µg/L. Inductively coupled plasma mass spectrometry (ICP-MS) was used to detect standard solution, test solution 1, and test solution 2. The test conditions were as follows: collection mode: KED, RF power: 1550.0W, cooling gas flow rate: 14.000L/min, atomization gas flow rate: 1.0600L/min, auxiliary gas flow rate: 0.8L/min, collision gas flow rate: 4.340ml/min. A standard curve was established based on the concentration of the test solution and the test results, and the concentration of yttrium ions in test solution 1 and test solution 2 was calculated based on the standard curve.

**Table 5 Phosphate washing**

| Name | Washing with phosphate |
|---|---|
| A | 120 ml of 30g/L sodium phosphate solution was added to the reaction vessel, stirred and mixed for 10 minutes, and discharged the liquid by filtration; then 150 ml of 10g/L sodium dihydrogen phosphate solution was added, stirred and mixed for 5 minutes, and discharged the liquid by filtration. |
| B | 120 ml of PBS solution with pH 7.4 was added to the reaction vessel, stirred and mixed for 10 minutes, and discharged the liquid by filtration; then 150 ml of PBS solution with pH 7.4 was added, stirred and mixed for 5 minutes, and discharged the liquid by filtration. |
| C | 120 ml of 30g/L sodium phosphate solution was added to the reaction vessel, stirred and mixed for 10 minutes, and discharged the liquid by filtration; then 150 ml of 10g/L sodium phosphate solution was added, stirred and mixed for 5 minutes, and discharged the liquid by filtration. |
| D | 120 ml of 30g/L sodium dihydrogen phosphate solution was added to the reaction vessel, stirred and mixed for 10 minutes, and discharged the liquid by filtration; then 150 ml of 10g/L sodium dihydrogen phosphate solution was added, stirred and mixed for 5 minutes, and discharged the liquid by filtration. |
| E | 120 ml of 30g/L sodium phosphate solution was added to the reaction |
| | vessel, stirred and mixed for 10 minutes, and discharged the liquid by filtration; then 150 ml of 10g/L disodium hydrogen phosphate solution was added, stirred and mixed for 5 minutes, and discharged the liquid by filtration. |

**Table 6 Statistics of yttrium ions concentration**

| Number | Yttrium ions added (mg/ml) | Yttrium ions in filtrate (mg/ml) | Yttrium ions precipitated (mg/ml) |
|---|---|---|---|
| B | 5.0 | 1.14×10⁻² | 1.85×10⁻⁶ |
| A | 5.0 | / | <1×10⁻⁶ |
| C | 5.0 | 2.22×10⁻⁵ | 2.51×10⁻⁶ |
| D | 2.0 | 1.54×10⁻⁵ | 1.98×10⁻⁶ |
| E | 3.5 | 8.57×10⁻⁶ | 2.92×10⁻⁶ |
| F | 5.0 | 5.95×10⁻⁶ | 3.44×10⁻⁶ |

| | | | |
|---|---|---|---|
| Note: / represents not detected. The lower limit of quantification is 1.00 ng/ml. | | | |

Due to previous experimental investigations, the methods of Group D and Group E were unable to adsorb a large amount of yttrium ions, resulting in a high concentration of yttrium ions in the filtrate and existing safety risk. Therefore, a 5mg/mL yttrium chloride [⁹⁰Y] solution was replaced with a 2mg/mL yttrium chloride [⁹⁰Y] solution and a 3.5mg/mL yttrium chloride [⁹⁰Y] solution to prepare yttrium [⁹⁰Y] microsphere injection. From the experimental results, it can be seen that even if the concentration of yttrium ions added (i.e. the concentration of yttrium chloride [⁹⁰Y] solution) was reduced, the concentration of yttrium ions and the concentration of precipitated yttrium ions in both groups of filtrates were still higher than the preferred solution A of the present application.

According to the above table, the amount of yttrium ions precipitation obtained in Group A experiment was significantly reduced, and no yttrium ions was detected in the filtrate. The resin microspheres prepared in Example 1 of the present application have good binding with yttrium ions, fewer yttrium ions precipitated, and significantly improved safety during preparation and use; however, other microspheres have poor binding with yttrium ions, and there are more yttrium ions in the filtrate.

Obviously, the above-mentioned embodiments are merely examples made for clear description, but do not limit the implementation. For those of ordinary skill in the art, other different forms of variations or modifications can also be made on the basis of the above-mentioned description. All embodiments are not necessary to be and cannot be exhaustively listed herein. In addition, the obvious variations or modifications derived therefrom all fall within the scope of protection of the present invention.

## Claims

1. An yttrium [⁹⁰Y] microsphere, wherein the yttrium [⁹⁰Y] microsphere comprises a polystyrene sulfonic acid type resin microsphere and yttrium-90 adsorbed within the polystyrene sulfonic acid type resin microsphere.

2. The yttrium [⁹⁰Y] microsphere of claim 1, wherein the polystyrene sulfonic acid type resin microsphere is prepared by reacting a polystyrene microsphere with a sulfonating agent.

3. The yttrium [⁹⁰Y] microsphere of claim 2, wherein the sulfonating agent is selected from one or more of liquid sulfur trioxide, concentrated sulfuric acid, chlorosulfonic acid, and sulfite.

4. The yttrium [⁹⁰Y] microsphere of claim 2, wherein the polystyrene microsphere is prepared by using styrene as a monomer and divinylbenzene as a crosslinking agent, mixing and reacting with an aqueous solution containing a suspending agent in the presence of an initiator and a pore forming agent.

5. The yttrium [⁹⁰Y] microsphere of claim 4, wherein the method for preparing the polystyrene sulfonic acid type resin microsphere comprises:
a preparation of polystyrene microspheres: mixing styrene, divinylbenzene, the initiator, and the pore forming agent, dissolving them to obtain an oil phase, injecting the oil phase into an aqueous solution containing a suspension agent, shearing emulsification, and heating reaction to obtain the polystyrene microspheres; and
a preparation of polystyrene sulfonic acid type resin microspheres: adding polystyrene sulfonic acid type resin microspheres to concentrated sulfuric acid, ultrasonic dispersing, and heating reaction to obtain the polystyrene sulfonic acid type resin microspheres.

6. The yttrium [⁹⁰Y] microsphere of claim 4 or 5, wherein the pore forming agent is selected from one or more of toluene and n-heptane; and/or, the suspension agent is polyvinyl alcohol; and/or, the initiator is benzoyl peroxide.

7. The yttrium [⁹⁰Y] microsphere of claim 6, wherein the polystyrene microsphere is made of the following raw materials:
an aqueous solution containing polyvinyl alcohol of 100 parts by volume;
syrene of 2.5 to 5.5 parts by volume;
divinylbenzene of 1.2 to 4.2 parts by volume;
benzoyl peroxide of 0.02-2.02 parts by weight;
toluene of 0.6 to 1.6 parts by volume; and
n-heptane of 0-1.6 parts by volume;
wherein the ratio between parts by weight and parts by volume is g/mL.

8. The yttrium [⁹⁰Y] microsphere of claim 1, wherein the yttrium [⁹⁰Y] microsphere has a radioactive activity ranging from 2.5 GBq/g to 25 GBq/g.

9. The yttrium [⁹⁰Y] microsphere of claim 1, wherein the polystyrene sulfonic acid type resin microspheres has a particle size ranging from 10µm to 60µm.

10. The yttrium [⁹⁰Y] microsphere of claim 9, wherein the polystyrene sulfonic acid type resin microspheres has a particle size ranging from 28µm to 43µm.

11. The yttrium [⁹⁰Y] microsphere of claim 10, wherein the polystyrene sulfonic acid type resin microspheres has a particle size ranging from 30µm to 35µm.

12. A method for preparing the yttrium [⁹⁰Y] microsphere of any one of claims 1 to 11, wherein the method comprises:
S1. mixing an yttrium chloride [⁹⁰Y]solution and yttrium sulfate-89 solution with polystyrene sulfonic acid type resin microspheres; and
S2. washing with a buffer salt solution and water after filtration to obtain an yttrium[⁹⁰Y] microsphere.

13. The method for preparing the yttrium [⁹⁰Y] microsphere of claim 12, wherein the ratio of radioactive activity of yttrium chloride [⁹⁰Y] solution, the volume of yttrium sulfate-89 solution, and the mass of polystyrene sulfonic acid type resin microspheres is 30-200GBq: 30-200 ml: 5-15.5 g.

14. The method for preparing the yttrium [⁹⁰Y] microsphere of claim 13, wherein the yttrium sulfate-89 solution has a concentration ranging from 1g/L to 9.5g/L.

15. The method for preparing the yttrium [⁹⁰Y] microsphere of claim 12, wherein the ratio of the volume ratio of the buffer salt solution to the mass of polystyrene sulfonic acid type resin microspheres is 70-300mL: 5-15.5 g.

16. The method for preparing the yttrium [⁹⁰Y] microsphere of claim 12, wherein the buffer salt solution is selected from one or more of sodium phosphate solution, sodium dihydrogen phosphate solution, disodium hydrogen phosphate solution, potassium phosphate solution, potassium dihydrogen phosphate solution, dipotassium hydrogen phosphate solution, PBS solution, sodium acetate and Tris-HCl buffer solution.

17. The method for preparing the yttrium [⁹⁰Y] microsphere of claim 16, wherein in the S2 step, sodium phosphate solution is used first for washing, and then sodium dihydrogen phosphate solution is used for washing.

18. The method for preparing the yttrium [⁹⁰Y] microsphere of claim 12, wherein in the S2 step, water, sodium phosphate solution, sodium dihydrogen phosphate solution, and water are used sequentially for washing.

19. The method for preparing the yttrium [⁹⁰Y] microsphere of claim 18, wherein the sodium dihydrogen phosphate solution has a concentration ranging from 2 g/L to 25g/L; the sodium phosphate solution has a concentration ranging from 10 g/L to 60g/L.

20. A pharmaceutical preparation of yttrium [⁹⁰Y] microsphere, wherein the pharmaceutical preparation comprises the yttrium [⁹⁰Y] microsphere of any one of claims 1 to 11 or yttrium [⁹⁰Y] microsphere prepared by the methods of any one of claims 12 to 19, and also a pharmaceutically acceptable excipient.

21. The pharmaceutical preparation of yttrium [⁹⁰Y] microsphere of claim 20, wherein the pharmaceutical acceptable excipient is a solvent.

22. The pharmaceutical preparation of yttrium [⁹⁰Y] microsphere of claim 20, wherein the pharmaceutical preparation is an injection, and the radioactive activity of an injection per unit dose is ranging from 3 GBq to 20GBq.

23. Use of the yttrium [⁹⁰Y] microsphere of any one of claims 1 to 11, or the yttrium [⁹⁰Y] microsphere as prepared by the methods of any one of claims 12 to 19, or pharmaceutical preparations of any one of claims 20 to 22 in the preparation of medicaments for preventing or treating liver cancer.
